# EUROPEAN PATENT APPLICATION

(11) **EP 2 607 885 A2**
(43) Date of publication of application: **26.06.2013**
(21) Application number: 12008567.5
(22) Date of filing: 21.12.2012
(51) Int. Cl.: G01N 21/35, G01J 3/453

(54) **Sample gas analyzing device and computer program for the same**

(30) Priority: 22.12.2011 JP 2011280640
(71) Applicant: HORIBA, LTD., Kyoto-shi, Kyoto 601-8510 (JP)
(72) Inventor: Itaya, Takahiro, Kyoto-shi, Kyoto, 601-8510 (JP); Nakatani, Shigeru, Kyoto-shi, Kyoto, 601-8510 (JP)
(74) Representative: Müller - Hoffmann & Partner

(57) **Abstract**

A sample gas analyzing device (100) that quantitatively analyzes one or more measurement target components in a sample gas by performing a multivariate analysis using a spectral spectrum obtained by irradiating light to the sample gas, wherein the analyzing device is adapted to switch the library data between a first generation condition in a period of a predetermined time lapse after starting the sample gas generation and a second generation condition after the predetermined time lapse, wherein under the first generation condition, a plurality of measurement target components are quantitatively analyzed using the first library data obtained by compensating interference influence of measurement extra-target components; and under the second generation condition, the quantitative analysis of a plurality of measurement target components is performed using second library data obtained without compensating interference influence of the measurement extra-target components.

## Description

### Technical Field

The present invention relates to an analyzing device that quantitatively analyzes measurement target components contained in a sample by performing a multivariate analysis using a spectral spectrum obtained by irradiating light to the sample, for example, using a method of Fourier-transform infrared spectroscopy (FTIR).

### Background Art

In a conventional gas analyzing device using the FTIR method, as disclosed in Patent Document 1, a comparative sample or a measurement sample is respectively accommodated in measurement cells and infrared light from an infrared light source is irradiated to the measurement cells so as to measure interferograms of the comparative samples or measurement sample. Then, these interferograms are respectively Fourier-transformed in an information processing unit so as to obtain power spectrums. Then, a ratio of the power spectrum of the measurement sample to the power spectrum of the comparative sample is calculated. This calculated ratio is then converted to an absorbance scale to thereby obtain an absorption spectrum. Then, components (single component or multiple components) contained in the measurement sample are quantitatively analyzed on the basis of the absorbance at wave number points in this absorption spectrum.

In this FTIR method, since there is a merit that a multi-component analysis of a measurement sample can be continuously and concurrently performed, this FTIR method is used in research and development of alternative fuels such as bio-ethanol mixed fuel and catalysts etc. in an engine exhaust gas field, and it is also used in evaluation of a reforming system by a concurrent analysis of methanol, carbon monoxide and carbon dioxide in a study of a fuel-cell methanol reforming system.

However, for example, in the case where a cold-start measurement is performed in an engine exhaust gas test, there is a problem that components other than measurement target components (for example, measurement extra-target components such as xylene, acetylene, propylene (or propane) and normal-hexane) are exhausted due to such as incomplete combustion of the fuel so that the measurement extra-target components exert interference influence on the measurement target components. Here, although it is conceivable to compensate the interference influence of the measurement extra-target components, the measurement extra-target components are not always exhausted in the cold-start measurement, and the measurement extra-target components are exhausted, for example, in a state that the catalyst has not been warmed up to a prescribed operating temperature (i.e., in an inactive state of the catalyst). If so, only a simple compensation of the interference influence of the measurement extra-target components in the cold-start measurement will results in an excessive compensation of the interference influence even in a state that the measurement extra-target components are no longer exhausted, and there arises a problem that a measurement error of the measurement target components becomes large.

### Citation List

### Patent Literature

Patent Document 1: JPA 2000-346801

### Summary of Invention

### Technical Problem

Therefore, the present invention has its essential object that reduction of the interference influence and reduction of the measurement error, which are in a trade-off relationship, can be made compatible in a quantitative analysis of one or more measurement target components in a sample.

### Solution to Problem

That is, a sample gas analyzing device pertaining to the present invention is configured to quantitatively analyze one or more measurement target components in a sample gas by performing a multivariate analysis using a spectral spectrum obtained by irradiating light to the sample gas. The sample gas analyzing device includes: library data including standard spectrum data for each of the one or more measurement target components for use in the multivariate analysis; and switching means adapted to switch the library data between a first generation condition which is a sample gas generation condition in a period of a predetermined time lapse after starting the sample gas generation and a second generation condition which is a sample gas generation condition after the predetermined time lapse, wherein, under the first generation condition, the quantitative analysis of the one or more measurement target components is performed using first library data obtained by compensating interference influence of measurement extra-target components which are components other than the measurement target components; and under the second generation condition, the quantitative analysis of the one or more measurement target components is performed using second library data obtained without compensating interference influence of the measurement extra-target components.

With this configuration, under the first generation condition where the interference influence of the measurement extra-target components with respect to the measurement target components largely appears, one or more measurement target components are quantitatively analyzed using the first library data obtained by compensating the interference influence of the measurement extra-target components. Whereas, under the second generation condition where the interference influence of the measurement extra-target components with respect to the measurement target components is small in an ignorable degree, one or more measurement target components are quantitatively analyzed using the second library data obtained without compensating the interference influence of the measurement extra-target components. Therefore, the reduction in interference influence and the reduction in measurement error can be made compatible.

In order that the effect of the present invention is made more remarkable, it is preferable that the sample is an engine exhaust gas, and the first generation condition is a period of a predetermined time lapse after starting the engine. The type of the gas components contained in the engine exhaust gas in a period of a predetermined time lapse (for example, a time period of a catalyst reaching a predetermined operating temperature, etc.) after starting the engine is different from that in a time period thereafter, and the interference influences thereof are also different. At this time, under the first generation condition, it is possible to compensate not only the interference influence among a plurality of measurement target components but also the interference influence due to various measurement extra-target components (for example, xylene, acetylene, propylene (or propane) and normal-hexane, etc.) generated by incomplete combustion of the fuel so that the interference influence can be reduced. Whereas, under the second generation condition, since the quantitative analysis of the measurement target components can be performed regardless of interference influence of the measurement extra-target components which are hard to be contained in the engine exhaust gas, the measurement error of the measurement target components can be reduced.

The measurement extra-target components contained in the sample gas are different according to the types of the fuel to be burned by the engine. Therefore, it is desirable that the sample gas analyzing device further includes a library data storage part for storing the first library data and the second library data, wherein the library data storage part stores the first library data respectively classified according to the types of the fuel to be burned by the engine. With this configuration, since the first library data is prepared for every type fuel, the interference influence of the measurement target components can be accurately compensated.

It is preferable that the sample gas analyzing device further includes a fuel type data reception part for receiving fuel type data indicating the types of the fuel, wherein the first library data corresponding to the types of the fuel received by the fuel type data reception part is used in the first generation condition. With this configuration, only by inputting fuel type data, the first library data corresponding to the type of the fuel can be automatically selected so that user's usability can be improved.

In order that the effect of the present invention is made more remarkable, it is preferable that the sample gas is a catalyst-passed gas produced by passing a simulated gas through a catalyst and that the first generation condition is a period of a predetermined time lapse after starting the passing of the simulated gas through the catalyst. Since the performance of the catalyst is varied in accordance with the temperature thereof, the type of the gas components contained in the catalyst-passed gas in a period of a predetermined time from the starting time of passing the simulated gas through the catalyst is different from the type thereof after that period, and the interference influence in the above period is also different from that thereafter. Thus, by differentiating the library data used under the first generation condition from that used under the second generation condition, the reduction in interference influence of the measurement target components and the reduction in measurement error can be made compatible.

In addition, a computer program pertaining to the present invention is used in a sample gas analyzing device that quantitatively analyzes one or more measurement target components in a sample gas by performing a multivariate analysis using a spectral spectrum obtained by irradiating light to the sample gas, the sample gas analyzing device including library data including standard spectrum data for each of the one or more measurement target components for use in the multivariate analysis, the program causing a computer to execute functions of: switching the library data between a first generation condition which is a sample gas generation condition in a period of a predetermined time lapse after starting the sample gas generation and a second generation condition which is a sample gas generation condition after the predetermined time lapse; under the first generation condition, performing the quantitative analysis of the one or more measurement target components using first library data obtained by compensating interference influence of measurement extra-target components which are components other than the measurement target components; and under the second generation condition, performing the quantitative analysis of the one or more measurement target components using second library data obtained without compensating interference influence of the measurement extra-target components.

### Advantageous Effects of Invention

According to the present invention configured as described above, by performing a specific compensation every generation condition in a quantitative analysis of one or more measurement target components in a sample, it becomes possible to improve the compensation accuracy of the measurement target components and the reduction in interference influence and the reduction in measurement error can be made compatible.

### Brief Description of Drawings

Fig. 1 is a schematic diagram showing a configuration of a sample gas analyzing device using the FTIR method of the present embodiment;
Fig. 2 is an equipment configuration diagram of a computing device of the same embodiment;
Fig. 3 is an equipment configuration diagram of a computing device of the same embodiment;
Fig. 4 is a schematic diagram showing gas components at a cold start and a library used in the cold start; and
Fig. 5 is a functional block diagram showing a computing device of a modified embodiment.

### Description of Embodiments

The following describes a sample gas analyzing device 100 using a FTIR method pertaining to the present invention with reference to the accompanying drawings.

The sample gas analyzing device 100 using the FTIR method of the present embodiment is intended for automobile exhaust gas to continuously measuring a multi-component concentration contained in the exhaust gas (sample gas) exhausted from an engine of the automobile.

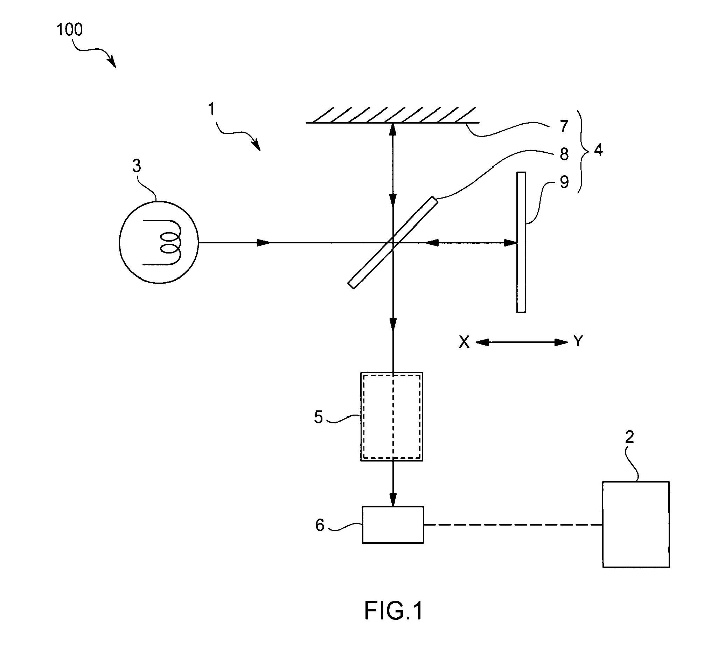

In specific, as shown in Fig. 1, this analyzing device 100 includes an analyzing part 1 which outputs an interferogram and a computing device 2 which processes the interferogram outputted from the analyzing part 1.

The analyzing part 1 includes: an infrared light source 3 configured to emit infrared light rays in parallel; an interference mechanism 4 interfering the infrared light rays from the infrared light source 3 to be outputted; a measurement cell 5 irradiated with the infrared light rays from the infrared light source 3 via the interference mechanism 4; and a semiconductor detector 6 for receiving the infrared light rays which have passed through the measurement cell 5. The interference mechanism 4 includes a fixed mirror 7, a beam splitter 8 and a movable mirror 9 which moves, for example, in parallel to the XY direction by a drive mechanism (not shown).

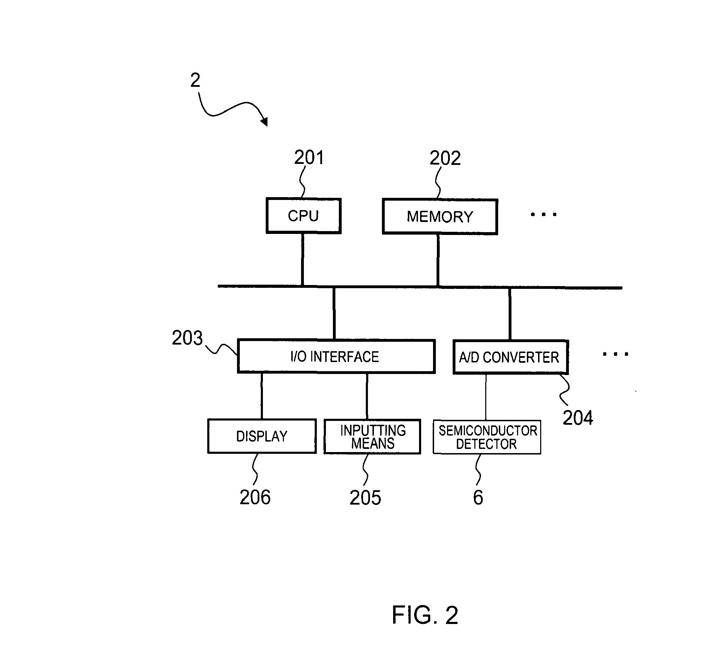

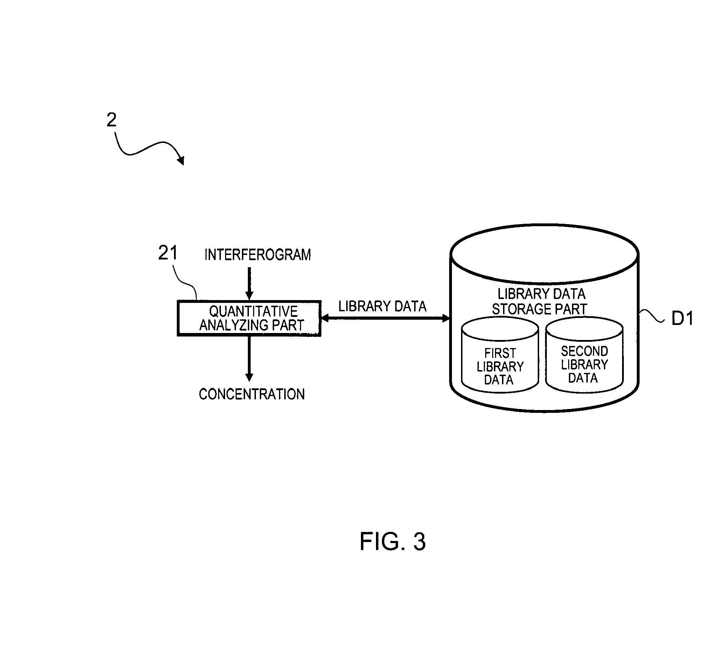

As shown in Fig. 2, the computing device 2 is a general-purpose or dedicated computer provided with a CPU 201, a memory 202, an I/O interface 203, an A/D converter 204, input means 205, a display and the like. This computer cooperates the CPU 201 and peripheral equipment and the like according to a prescribed program stored in a predetermined region of the memory 202 so as to exhibit functions as the library storage part D1, the quantitative analyzing part 21 etc. as shown in Fig. 3.

The library data storage part D1 stores library data including known standard spectral data for each of a plurality of measurement target components (for example, ethanol, water, formaldehyde, etc.) for use in multivariate analysis and calibration curve data and the like.

In specific, the library storage part D1 stores the first library data corresponding to the first generation condition (0 ≤ t ≤ Tx) which is an exhaust gas generation condition from a starting time of an engine up to a predetermined time Tx lapse in an engine exhaust gas test and the second library data corresponding to the second generation condition (t > Tx) which is an exhaust gas generation condition after the predetermined time Tx lapse.

The first library data is library data including standard spectral data obtained by compensating interference influence of the measurement extra-target components (i.e., components other than a plurality of measurement target components, for example, xylene, acetylene, propylene (or propane) and normal-hexane, etc.). Meanwhile, the second library data is library data including standard spectral data obtained without compensating the interference influence of the measurement extra-target components. These first library data and second library data are previously calculated and stored in the library data storage part D1.

The quantitative analyzing part 21 receives an interferogram outputted from the semiconductor detector 6 of the analyzing part 1 and acquires the first library data or the second library data and calibration curve data etc. from the library data storage part D1 so as to calculate the concentration of each of the measurement target components. In specific, an interferogram of a comparative sample and an interferogram of a measurement sample are respectively Fourier-transformed so as to obtain power spectrums. Then, a ratio of the power spectrum of the measurement sample to the power spectrum of the comparative sample is calculated. This calculated ratio is then converted to an absorbance scale to thereby obtain an absorption spectrum. Then, the concentrations of the measurement target components (single component or multiple components) contained in the measurement sample are calculated on the basis of the absorbance at a plurality of wave number points in this absorption spectrum.

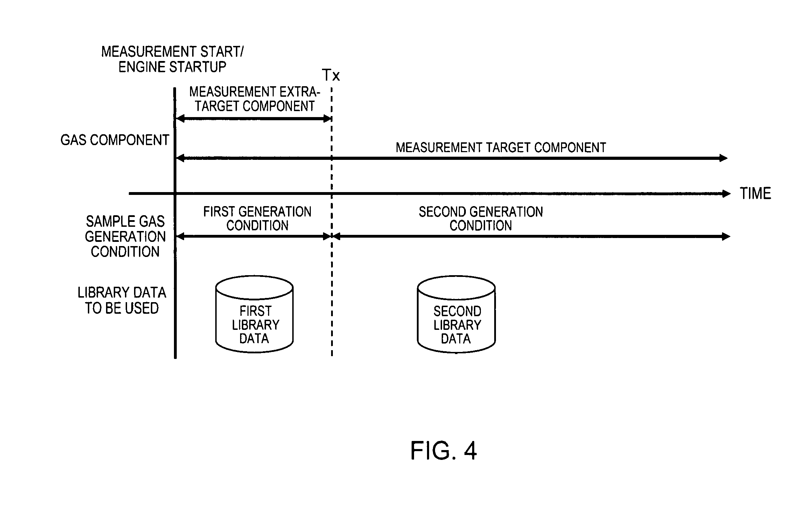

In specific, in the case where a cold-start measurement is performed in an exhaust gas test, the quantitative analyzing part 21 detects that the engine has started and measures the time lapse after the engine has started. Then, as shown in Fig. 4, during a predetermined time Tx after the starting of the engine, the quantitative analyzing part 21 acquires the first library data from the library data storage part D1 and compensates mutual interference influence of the measurement target components and interference influence of the measurement extra-target components so as to calculate the concentration of the measurement target components. On the other hand, after the predetermined time Tx from the starting of the engine, the quantitative analyzing part 21 acquires the second library data from the library data storage part D1 and compensates mutual interference influence of the measurement target components so as to calculate the concentration of the measurement target components. Here, the predetermined time Tx mentioned above is a time required for a temperature of a catalyst provided, for example, in a tail pipe to reach a desired temperature so that the catalyst becomes an active state.

In this configuration, when determining the first generation condition, the quantitative analyzing part 21 determines that the condition is the first generation condition in the case where the stopped state of the engine before starting the engine is continued for a predetermined time. Whereas, the quantitative analyzing part 21 determines that the condition is not the first generation condition but the second generation condition in the case where the stopped state of the engine is continued no longer than a predetermined time. It is noted that a predetermined time referred to here is the time that, for example, the temperature of the catalyst after stopping the engine is lowered so that the catalyst becomes in an inactive state.

It is noted that, although the switching of the library data acquisition between the first library data and the second library data is performed by measuring the time elapsed after starting the engine in this example, it is also considered that the switching may be performed by following methods. For example, it may be considered that the quantitative analyzing part 21 (1) acquires a rotational engine speed signal and switches when the rotational engine speed satisfies a predetermined condition, (2) acquires a temperature detection signal from a temperature sensor detecting a temperature of the engine exhaust gas and switches when the temperature of the engine exhaust gas becomes equal to or higher than a predetermined temperature, (3) acquires a flow rate detection signal from a flow rate sensor detecting a flow rate of the engine exhaust gas and switches when the flow rate of the engine exhaust gas becomes equal to or larger than a predetermined flow rate, (4) acquires a catalyst temperature detection signal from a catalyst temperature sensor detecting a temperature of the catalyst provided in a tail pipe (exhaust pipe) and switches when the catalyst temperature becomes equal to or higher than a predetermined temperature (a temperature indicative of an active state), or (5) acquires an engine temperature signal from an engine temperature sensor detecting an engine temperature and switches when the engine temperature becomes equal to or higher than a predetermined temperature, etc.. In addition, the quantitative analyzing part 21 may also be configured to switch by applying a time constant on the detection signal or may be configured to switch by comparing a magnitude of a movement average of such as detection temperature and detection flow rate with respect to a threshold value.

According to the gas analyzing device 100 pertaining to the present embodiment configured like this, under the first generation condition where the interference influence of the measurement extra-target components with respect to the measurement target components largely appears, one or more measurement target components are quantitatively analyzed using the first library data obtained by compensating the interference influence of the measurement extra-target components. Whereas, under the second generation condition where the interference influence of the measurement extra-target components with respect to the measurement target components is small in an ignorable degree, one or more measurement target components are quantitatively analyzed using the second library data obtained without compensating the interference influence of the measurement extra-target components. Therefore, the reduction in interference influence and the reduction in measurement error can be made compatible. In particular, since the interference influence of the measurement extra-target components is compensated under the first generation condition, it is possible to compensate negative interference and positive interference due to the measurement extra-target components that are easily generated under the first generation condition.

In addition, since the quantitative analyzing part 21 is configured to automatically switch between the first library data and the second library data, an optimum compensation can be performed in accordance with the generation condition while quantitatively analyzing the engine exhaust gas similarly to a normal measurement.

### <Other Modified Embodiment>

It should be noted that the present invention is not limited to the above embodiment.

For example, although the sample gas analyzing device using the FTIR method for automobile exhaust gas is described in the above embodiment, the present invention can be used for various other applications such as a sample gas analyzing device using a FTIR method for a reforming system of fuel cell methanol. In addition, the present invention can be also applied to an analyzing device using an ICP light emission analyzing method or an analyzing device in which measurement components are affected by interference components such as an analyzing device using a Raman spectroscopy.

In addition, the sample gas analyzing device of the present invention can be also used in combination with a catalyst evaluation device that generates a simulated exhaust gas for performing an evaluation test of a catalyst and passes the simulated exhaust gas through the catalyst to be tested. In this case, the sample gas is catalyst-passed gas which has passed through the catalyst and the first generation condition is a predetermined time elapsed from a start up of the simulated exhaust gas passing through the catalyst.

Furthermore, although the quantitative analyzing part is configured to automatically switch a plurality of types of library data in the above embodiment, the library data may be manually switched by a user.

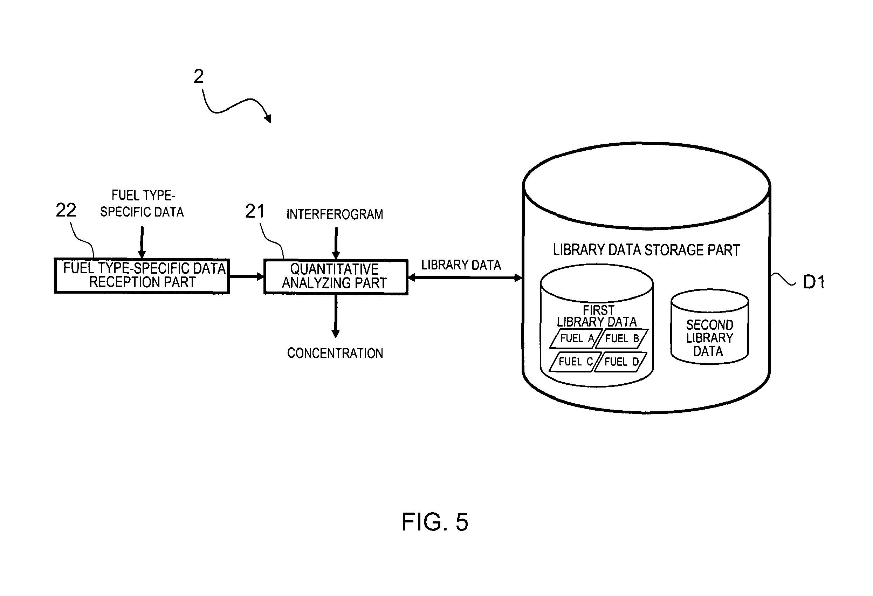

Moreover, the types of the measurement extra-target components contained in the engine exhaust gas are also different in accordance with the types of the fuel to be burned in the engine under the first generation condition. For example, in the case of using gasoline or diesel fuel, propylene is a measurement target component and propane is a measurement extra-measurement component. However, in the case of using CNG (compressed natural gas), propane is a measurement target component and propylene is a measurement extra-measurement component. As the other fuels, alcohol fuel and dimethyl ether etc. may be considered. Therefore, the first library data to be stored in the library data storage part D1 may be prepared in separation by types of the fuels to be burned by an engine. In addition, Fig. 5 shows a case of preparing the library data as the first library data for each of fuel types A to D. With this arrangement, since the first library data is prepared for each type of fuels, the interference influence of the measurement target components under the first generation condition can be accurately compensated every type of fuels.

In this case, as shown in Fig. 5, the computing device 2 of the sample gas analyzing device 100 includes a fuel type data reception part 22 for receiving fuel type data indicative of a fuel type, and it is considered that the quantitative analyzing part 21 is configured to acquire the first library data corresponding to a type of the fuel indicated by the fuel type data from the library data storage part D1 on the basis of the fuel type data received by the fuel type data reception part 22. With this configuration, only by inputting the fuel type data by a user, the first library data corresponding to the type of the fuel can be automatically selected so that the usability of the user can be improved.

Moreover, although the library data of the above embodiment is prepared for each of the first generation condition and the second generation condition by separating the cold-start measurement into the first generation condition and the second generation condition with time lapse, it may be also possible to prepare a plurality of types of library data by differentiating the measurement method and measurement target etc. so that each of the different measurement methods and measurement targets is used as each of the generation conditions.

In addition, under the first generation condition, other than calculating the concentration of the measurement target components obtained by compensating the interference influence of the measurement extra-target components with respect to the measurement target components, it may be configured that the concentration of the measurement extra-target components is calculated. In this case, the first library data includes standard spectral data of the measurement extra-target components.

In addition, the present invention should not be limited to the embodiment described above, and various modifications are of course possible within the scope unless departing from the intended spirit thereof.

### Reference Signs List

- 100: ... Sample gas analyzing device
- D1: ... Library data storage part
- 21: ... Quantitative analyzing part
- 22: ... Fuel type data reception part

## Claims

1. A sample gas analyzing device that quantitatively analyzes one or more measurement target components in a sample gas by performing a multivariate analysis using a spectral spectrum obtained by irradiating light to the sample gas, comprising:
library data including standard spectrum data for each of the one or more measurement target components for use in the multivariate analysis; and
switching means adapted to switch the library data between a first generation condition which is a sample gas generation condition in a period of a predetermined time lapse after starting the sample gas generation and a second generation condition which is a sample gas generation condition after the predetermined time lapse, wherein
under the first generation condition, the quantitative analysis of the one or more measurement target components is performed using first library data obtained by compensating interference influence of measurement extra-target components which are components other than the measurement target components; and
under the second generation condition, the quantitative analysis of the one or more measurement target components is performed using second library data obtained without compensating interference influence of the measurement extra-target components.

2. The sample gas analyzing device according to claim 1, wherein the sample gas is an engine exhaust gas exhausted from an engine, and wherein the first generation condition is a period of a predetermined time lapse after starting the engine.

3. The sample gas analyzing device according to claim 2 further comprising a library data storage part for storing the first library data and the second library data, wherein the library data storage part stores the first library data respectively classified according to types of fuel to be burned by the engine.

4. The sample gas analyzing device according to claim 3 further comprising a fuel type data reception part for receiving fuel type data indicating the types of the fuel, wherein the first library data corresponding to the types of fuel received by the fuel type data reception part is used in the first generation condition.

5. The sample gas analyzing device according to claim 1, wherein the sample gas is a catalyst-passed gas produced by passing a simulated gas through a catalyst, and wherein the first generation condition is a period of a predetermined time lapse after starting the passing of the simulated gas through the catalyst.

6. A computer program for use in a sample gas analyzing device that quantitatively analyzes one or more measurement target components in a sample gas by performing a multivariate analysis using a spectral spectrum obtained by irradiating light to the sample gas, the sample gas analyzing device including library data including standard spectrum data for each of the one or more measurement target components for use in the multivariate analysis, the program causing a computer to execute functions of:
switching the library data between a first generation condition which is a sample gas generation condition in a period of a predetermined time lapse after starting the sample gas generation and a second generation condition which is a sample gas generation condition after the predetermined time lapse;
under the first generation condition, performing the quantitative analysis of the one or more measurement target components using first library data obtained by compensating interference influence of measurement extra-target components which are components other than the measurement target components; and
under the second generation condition, performing the quantitative analysis of the one or more measurement target components using second library data obtained without compensating interference influence of the measurement extra-target components.
